# EUROPEAN PATENT APPLICATION

(11) **EP 3 799 074 A1**
(43) Date of publication of application: **31.03.2021**
(21) Application number: 19200492.7
(22) Date of filing: 30.09.2019
(51) Int. Cl.: G16H 50/70

(54) **HEALTHCARE NETWORK**

(71) Applicant: Siemens Healthcare GmbH, 91052 Erlangen (DE)
(72) Inventor: Frings, Oliver, 91058 Erlangen (DE); Boettger, Thomas, 91054 Erlangen (DE); Hartung, Ulrich, 91094 Langensendelbach (DE); Kartmann, René, 90491 Nürnberg (DE); Roth, Dorothee, 91054 Erlangen (DE); Krüger, Benedikt, 96250 Ebensfeld (DE); Kubala, Eugen, 80939 München (DE); Neumann, Dominik, 91052 Erlangen (DE); Würstle, Maximilian, 91083 Baiersdorf (DE)

(57) **Abstract**

A system (100) operable to transmit healthcare data to a user device (106a-d) is provided. The user device (106a-d) is configured for use in analysing medical information. The system (100) maintains data representing a first directed graph (600), which represents at least part of a medical guideline, in a database (108) and also maintains a plurality of patient models comprising healthcare data in a database (108). The system (100) selects an element from the first directed graph (600) by processing the plurality of patient models and the data representing the first directed graph (600). Based on a combination of the selected element and the plurality of patient models, a first patient cohort and a second patient cohort are identified, treatment of the first patient cohort having deviated from the at least part of a medical guideline at the selected element. At least one patient cohort characteristic which distinguishes the first patient cohort from the second patient cohort is determined by processing the plurality of patient models. A second directed graph (610, 620) is generated, based on at least the at least one identified patient cohort characteristic, and transmitted for receipt by the user device (106a-d).

## Description

Embodiments described herein relate generally to providing healthcare data to a user device. More specifically the embodiments relate to methods, systems, and computer programs for transmitting healthcare data to a user device configured for use in analysing medical information.

Medical guidelines provide recommendations for how people with specific medical conditions should be treated. Medical guidelines may indicate which diagnostic or therapeutic steps should be taken when treating a patient with a specific condition and what follow-up procedures should be performed dependent on the results of the diagnostic or therapeutic steps. Some medical guidelines provide information about the prevention, prognosis of certain medical conditions as well as the risk and/or benefits, and take in account the cost-effectiveness associated with diagnostic and therapeutic steps in the treatment of a patient. The information contained within a guideline is generally specific to a particular medical domain.

Data pertaining to patients being treated for a medical condition are typically generated during diagnostic and therapeutic steps. This data is typically stored in disparate sources relating to the locations, such as clinical centres or hospitals, in which the data is generated. Data pertaining to patients may be encoded to relate the raw data or values with the respective clinical steps which generated said data. Data may be encoded using clinical coding systems such as SNOMED CT, LOINC, Siemens® internal coding system, among other coding systems.

Patient conditions and diseases do not always conform with recommendations and clinical pathways provided in Medical guidelines. A clinical pathway, also called a disease pathway, may include secondary prevention, screening, diagnostics, diagnosis, therapy decisions, therapy and follow-up treatments or decisions. As such, a medical guideline alone may not always be sufficient to enable sufficient analysis.

A system for transmitting healthcare data for receipt by a user device is described in the European Patent Application EP18199915 filed on 11 Oct 2018, and in the European Patent Application EP18208021 filed on 23 Nov 2018, which are hereby incorporated by reference.

### Summary

According to a first aspect of the present invention, there is provided a system operable to transmit healthcare data to a user device, the user device being configured for use in analysing medical information, the system comprising at least one processor and at least one memory including computer program code, the at least one memory and computer program code configured to, with the at least one processor, cause the system to: maintain, in a first database, data representing a first directed graph representing at least part of a medical guideline, the first directed graph comprising a plurality of elements representing a clinical step; maintain, in a second database, a plurality of patient models each comprising healthcare data associated with a respective patient; select at least one element from the plurality of elements by processing the plurality of patient models and the data representing the first directed graph to identify at least one element at which treatment of a subset of patients has deviated from the at least part of a medical guideline; identify, based on a combination of the at least one selected element and the plurality of patient models, a first patient cohort whose treatment has deviated from the at least part of a medical guideline at the at least one selected element and a second patient cohort whose treatment has conformed to the at least part of a medical guideline at the at least one selected element; process the plurality of patient models representing the first and second patient cohorts to identify at least one patient cohort characteristic distinguishing the first patient cohort from the second patient cohort; generate a second directed graph dependent at least on the at least one identified patient cohort characteristic; and transmit data representing the second directed graph for receipt by the user device.

In this way the system may be operable to identify patient cohorts for which treatment as specified in at least part of a medical guideline may not, be followed. In these cases, the system may then generate a second directed graph which comprises an indication of a deviation from the medical guideline for the identified patient cohort. This is used to guide the decisions of medical practitioners so that they can provide more effective treatment to patients who belong to a specific patient cohort for which the medical guideline may not be effective if adhered to.

Selecting the at least one element may comprise processing the plurality of patient models and the data representing the first directed graph to identify at least one element at which the subset of patients whose treatment has deviated from the at least part of the medical guideline exceeds a predetermined proportion of the patients associated with the plurality of patient models. This may provide appropriate selection of the at least one element in a variety of situations where the total number patient models available differs.

The at least one patient cohort characteristic may comprise at least one of: an age; a height; a weight; a sex; a body mass index; a genetic mutation; an associated medical practitioner; and a location.

The memory and computer program code may be configured to, with the at least one processor, cause the system to override the selection of the at least one element based on a user input. In this way, a user, such as a medical practitioner, of the system may identify an element for which they suspect a deviation may be occurring for a patient cohort or for which they suspect a deviation from the at least part of a medical guideline for a certain patient cohort may be beneficial.

The at least one selected element may be associated with at least one conditional parameter value, the patient models may each comprise a plurality of patient attribute values corresponding to respective clinical steps, and the first and second patient cohorts may be identified based on a comparison of the at least one conditional parameter value with respective patient attribute values from the plurality of patient models. This may, for example, provide an efficient and robust way of automatically identifying the first and second patient cohorts.

The first and second patient cohorts may be identified based on an availability of healthcare data in the respective patient models corresponding to the at least one selected element. This may allow the system to quickly identify whether a patient as conformed to at least part of the medical guideline.

Processing the plurality of patient models representing the first and second patient cohorts to determine at least one patient cohort characteristic distinguishing the first patient cohort from the second patient cohort may comprise processing the plurality of patient models using at least one of: principal component analysis; random forest regression; and regularized regression. These methods may provide efficient and accurate ways of identifying distinguishing patient cohort characteristics from a large number of patient characteristics associated with each patient.

The second directed graph may comprise an indication that the clinical step represented by the at least one selected element is not recommended for patients associated with the at least one identified patient cohort characteristic. This may allow a medical practitioner providing treatment to a patient based on the second directed graph to be alerted to ways in which they can provide more effective treatment to their patients. This may, for example, allow physicians who do not have experience treating patients belonging to a particular patient cohort to provide tailored healthcare which may not be covered in the associated medical guideline.

The second directed graph may comprise: a plurality of nodes; a set of directed edges; and at least one further node connected to the selected element, wherein the at least one further node may comprise the indication that a clinical step represented by the selected element is not recommended for patients associated with the at least one identified patient cohort characteristic. In this way, a medical practitioner may be alerted to a potential deviation in treatment for patients belonging to a specific patient cohort.

The second directed graph may comprise at least one further directed edge connected at a first end to the further node, the further directed edge may be indicative of a deviation from the at least part of a medical guideline for the first patient cohort.

The at least one memory and computer program code may be configured to, with the at least one processor, cause the system to: based on a user input indicative of a decision in respect of patients associated with the at least one identified patient cohort characteristic, modify the second directed graph. This may allow the system to automatically generate different treatment pathways, including further clinical steps, in the second directed graph based on medical decisions made with respect of patients belonging to the first patient cohort. This can identify new and/or beneficial treatment pathways for specific patient cohorts which are, as of yet, not specified in a medical guideline.

The at least one memory and computer program code may be configured to, with the at least one processor, cause the system to: maintain, in a third database, a further patient model comprising healthcare data associated with a patient, the patient being associated with the at least one identified patient cohort characteristic; determine, based on a combination of the second directed graph and the further patient model, a status of the at least one further node and/or a status of a directed edge connected thereto; and dependent on the status of the at least one further node and/or the status of the directed edge connected thereto, transmit data indicative of the status of the at least one further node and/or directed edge connected thereto for receipt by the user device, the data indicative of the status of the at least one further node and/or directed edge connected thereto being for use in determining whether a clinical step represented by the selected node is not recommended for the patient.

The system may automatically map a further patient model to the second directed graph and, if the treatment of the patient represented by this further patient model is at or approaching a node for which treatment of patients, belonging to a specific cohort, have been known to deviate, the system may alert a user.

The at least one memory and computer program code may be configured to, with the at least one processor, cause the system to: dependent on the status of the further node and/or the status of the directed edge connected thereto, transmit data indicative of a request for input from a user of the user device for receipt by the user device; receive, from the user device, data indicative of further clinical steps to be represented by a further plurality of nodes; and modify the second directed graph based on the received data indicative of the further clinical steps. Once a deviation from the at least part of a medical guideline for a specific patient cohort has been identified, the system may be operable to generate new nodes representing alternative clinical steps, following the deviation, as specified by medical practitioners. Physicians can manually alter the second directed graph to reflect their preferred treatment pathways following the deviation.

According to a second aspect of the present invention, there is provided a computer program comprising a set of instructions, which, when executed by a computerised device, cause the computerised device to perform a method of transmitting healthcare data to a user device, the user device being configured for use in analysing medical information, the method comprising: maintaining, in a first database, data representing a first directed graph representing at least part of a medical guideline, the first directed graph comprising a plurality of elements representing a clinical step; maintaining, in a second database, a plurality of patient models each comprising healthcare data associated with a respective patient; selecting at least one element from the plurality of elements by processing the plurality of patient models and the data representing the first directed graph to identify at least one element at which treatment of a subset of patients has deviated from the at least part of a medical guideline; identifying, based on a combination of the at least one selected element and the plurality of patient models, a first patient cohort whose treatment has deviated from the at least part of a medical guideline at the at least one selected element and a second patient cohort whose treatment has conformed to the at least part of a medical guideline at the at least one selected element; processing the plurality of patient models representing the first and second patient cohorts to identify at least one patient cohort characteristic distinguishing the first patient cohort from the second patient cohort; generating a second directed graph dependent at least on the at least one identified patient cohort characteristic; and transmitting data representing the second directed graph for receipt by the user device.

According to a third aspect of the present invention, there is provided a method of transmitting healthcare data to a user device, the user device being configured for use in analysing medical information, the method comprising: maintain, in a first database, data representing a first directed graph representing at least part of a medical guideline, the first directed graph comprising a plurality of elements representing a clinical step; maintaining, in a second database, a plurality of patient models each comprising healthcare data associated with a respective patient; selecting at least one element from the plurality of elements by processing the plurality of patient models and the data representing the first directed graph to identify at least one element at which treatment of a subset of patients has deviated from the at least part of a medical guideline; identifying, based on a combination of the at least one selected element and the plurality of patient models, a first patient cohort whose treatment has deviated from the at least part of a medical guideline at the at least one selected element and a second patient cohort whose treatment has conformed to the at least part of a medical guideline at the at least one selected element; processing the plurality of patient models representing the first and second patient cohorts to identify at least one patient cohort characteristic distinguishing the first patient cohort from the second patient cohort; generating a second directed graph dependent at least on the at least one identified patient cohort characteristic; and transmitting data representing the second directed graph for receipt by the user device.

According to a fourth aspect of the present invention, there is provided a system operable to transmit healthcare data to a user device, the user device being configured for use in analysing medical information, the system comprising at least one processor and at least one memory including computer program code, the at least one memory and computer program code configured to, with the at least one processor, cause the system to: maintain, in a first database, data representing a first directed graph representing at least part of a medical guideline and a second directed graph representing the at least part of a medical guideline and a modification to the at least part of a medical guideline, each directed graph comprising a respective plurality of elements representing a clinical step; maintain, in a second database, a plurality of patient models each comprising healthcare data associated with a respective patient; identify a first set of the patient models representing patients that have been treated based on the at least part of a medical guideline as represented by the first directed graph and a second set of the patient models representing patients that have been treated based on the at least part of a medical guideline as represented by the second directed graph; determine, based on a comparison of the first set of patient models with the second set of the patient models, which of the first and second directed graphs is a preferred directed graph; and responsive to the determination, transmit data representing the preferred directed graph for receipt by the user device.

Directed graphs may be generated in a plurality of ways and in some cases, custom directed graphs which comprise changes to the treatment pathways may be generated automatically based on past treatment of patients, or manually, for example, based on hypotheses of medical practitioners. This aspect of the present invention may allow the effectiveness, with respect to treatment of patients, of such directed graphs to be compared. The future treatment of patients may then use a preferred directed graph to provide medical care to patients.

The modification to the at least part of a medical guideline is represented by at least one of: at least one node in the second directed graph; and at least one directed edge in the second directed graph. This may, for example, provide differences between the first and second directed graphs which can be directly compared using patient models.

The plurality of patient models may each comprise a plurality of patient entries, at least one of the patient entries may include at least one patient outcome measure, and determining which of the first and second directed graphs is a preferred directed graph may comprise comparing a first plurality of patient outcome measures of the first set of patient models with a second plurality of patient outcome measures of the second set of patient models. Using patient outcome measures to compare the effectiveness of treatment provided based on the first and second directed graphs, may ensure that the directed graph which is selected as the preferred directed graph provides better patient outcomes than the other directed graph when used to treat patients.

The modification of the at least part of a medical guideline may be associated with a patient cohort characteristic and the first and second sets of patient models may be identified based on the patient cohort characteristic. In this way the effectiveness of directed graphs generated based on deviations for specific patient cohorts, as described above in relation to the first aspect of the present invention, may be tested.

The at least one memory and computer program code may be configured to, with the at least one processor, cause the system to identify the first set of patient models based on a comparison of the plurality of patient models and the first directed graph, wherein comparing the plurality of patient models with the first directed graph may comprise, for each patient model, determining a status of at least one of the plurality of elements of the first directed graph.

The at least one memory and computer program code may be configured to, with the at least one processor, cause the system to identify the second set of patient models based on a comparison of the plurality of patient models and the second directed graph, wherein comparing the plurality of patient models with the second directed graph may comprise, for each patient model, determining a status of at least one of the plurality of elements of the second directed graph. This may provide a robust and reliable way to identify patients represented by the second set of patients based on available data. This may alleviate the need to manually select which patients have been treated according to which guideline.

For a said patient model, the status of a said element may be dependent on availability of data, associated with a clinical step which is associated with the said element, in the said patient model. If a patient has not undergone a particular clinical step, then their associated patient model will not comprise data associated with the clinical step. This allows such patients to be efficiently identified.

Each patient model may comprise a plurality of patient entries, each patient entry comprising at least one attribute value, and determining a status of a said element may comprise: maintaining a first association between at least one of the patient entries of a said patient model and an identifier from a plurality of identifiers; maintaining a second association between the said element and the identifier from the plurality of identifiers; selecting, based on the first and second association, a said attribute value associated with the said element; and determining, based on a comparison of the attribute value associated with the said element to a conditional parameter value associated with the said element, whether the conditional parameter value associated with the said element is satisfied.

Determining which of the directed graphs is a preferred directed graph may comprise: determining a first measure indicative of an average conformity of the first set of patient models to the first directed graph; determining a second measure indicative of an average conformity of the second set of patient models to the second directed graph; and performing a comparison of the first plurality of patient outcome measures with the second plurality of patient outcome measures using the first and second measures. In this way, the selection of a preferred directed graph may be sensitive to the general adherence of patients to said directed graph. For example, although a given directed graph may have average or good outcome measures it may be that the general adherence to the second directed graph is poor, and therefore treatment according to said directed graph may be less predictable and/or more variable. By taking this into account when selecting a directed graph as a preferred directed graph one can control this characteristic to an extent.

The first measure may be dependent on an average status for the first set of patient models of the plurality of elements of the first directed graph. This may provide a measure of the average adherence which can be used as a variable in the selection of a preferred directed graph.

Similarly, the second measure may be dependent on an average status for the second set of patient models of the plurality of elements of the second directed graph.

Determining which of the first and second directed graphs is a preferred directed graph may comprise: transmitting data indicative of a result of the comparison of the first set of patient models with the second set of patient models for receipt by the user device; receiving from the user device data indicative of a decision in respect of the result of the comparison; and selecting, based on the received data indicative of the decision, one of the first or second directed graphs. In this way, a user may be able to select a preferred directed graph based on a comparison of the outcome measures. In some examples, comparing the outcome measures may not be a simple process of comparing one set of variables to another, for example where the variables are interrelated and/or non-linear, or where some variables are of more importance than others. In this case, presenting a result of the comparison to a user may allow the user to resolve the selection of a preferred directed graph.

The at least one memory and computer program code may be configured to, with the at least one processor, cause the system to: if the first directed graph is the preferred directed graph, transmit data indicative of the first plurality of patient outcome measures for receipt by the user device; or if the second directed graph is the preferred directed graph, transmit data indicative of the second plurality of patient outcome measures for receipt by the user device. In this way, a user of a user device may be provided with supplementary medical information to support the decision for using the preferred directed graph for, for example, discussing with patients why a specific treatment pathway is being used and/or to justify to the medical practitioner that the preferred directed graph enables the better treatment for their patients.

According to a fifth aspect of the present invention, there is provided a computer program comprising a set of instructions, which, when executed by a computerised device, cause the computerised device to perform a method of transmitting healthcare data to a user device, the user device being configured for use in analysing medical information, the method comprising: maintaining, in a first database, data representing a first directed graph representing at least part of a medical guideline and a second directed graph representing the at least part of a medical guideline and a modification to the at least part of a medical guideline, each directed graph comprising a respective plurality of elements representing a clinical step; maintaining, in a second database, a plurality of patient models each comprising healthcare data associated with a respective patient; identifying a first set of the patient models representing patients that have been treated based on the at least part of a medical guideline as represented by the first directed graph and a second set of the patient models representing patients that have been treated based on the at least part of a medical guideline as represented by the second directed graph; determining, based on a comparison of the first set of the patient models with the second set of the patient models, which of the first and second directed graphs is a preferred directed graph; and responsive to the determination, transmitting data representing the preferred directed graph for receipt by the user device.

According to a sixth aspect of the present invention, there is provided a method of transmitting healthcare data to a user device, the user device being configured for use in analysing medical information, the method comprising: maintaining, in a first database, data representing a first directed graph representing at least part of a medical guideline and a second directed graph representing the at least part of a medical guideline and a modification to the at least part of a medical guideline, each directed graph comprising a respective plurality of elements representing a clinical step; maintaining, in a second database, a plurality of patient models each comprising healthcare data associated with a respective patient; identifying a first set of the patient models representing patients that have been treated based on the at least part of a medical guideline as represented by the first directed graph and a second set of the patient models representing patients that have been treated based on the at least part of a medical guideline as represented by the second directed graph; determining, based on a comparison of the first set of the patient models with the second set of the patient models, which of the first and second directed graphs is a preferred directed graph; and responsive to the determination, transmitting data representing the preferred directed graph for receipt by the user device.

### Brief Description of Drawings

Figure 1 shows a schematic block diagram of an example system in accordance with embodiments;
Figure 1a shows a schematic block diagram of an example system connected to a network in accordance with embodiments;
Figure 2 shows an example of a directed graph in accordance with embodiments;
Figure 3 shows a schematic block diagram of an event model in accordance with embodiments;
Figure 4 shows a schematic block diagram of a patient model in accordance with embodiments;
Figure 5 shows a flow chart of an operation of the system in accordance with embodiments;
Figures 6A to 6C show examples of a directed graph comprising an indication in accordance with embodiments;
Figures 7A to 7C show plots of patient models in a feature space to graphically represent a determination of at least one patient cohort characteristic in accordance with embodiments; and
Figure 8 shows a flow chart of an operation of the system in accordance with embodiments.

### Detailed Description

Embodiments will now be described in the context of systems, methods, and computer programs for providing information to a user of a user device, the device being configured for use in analysing medical information. Reference will be made to the accompanying drawings. In the following description, for the purpose of explanation, numerous specific details of certain examples are set forth. Reference in the specification to "an example" or similar language means that a particular feature, structure, or characteristic described in connection with the example is included in at least that one example, but not necessarily in other examples. It should further be noted that certain examples are described schematically with certain features omitted and/or necessarily simplified for ease of explanation and understanding of the concepts underlying the examples.

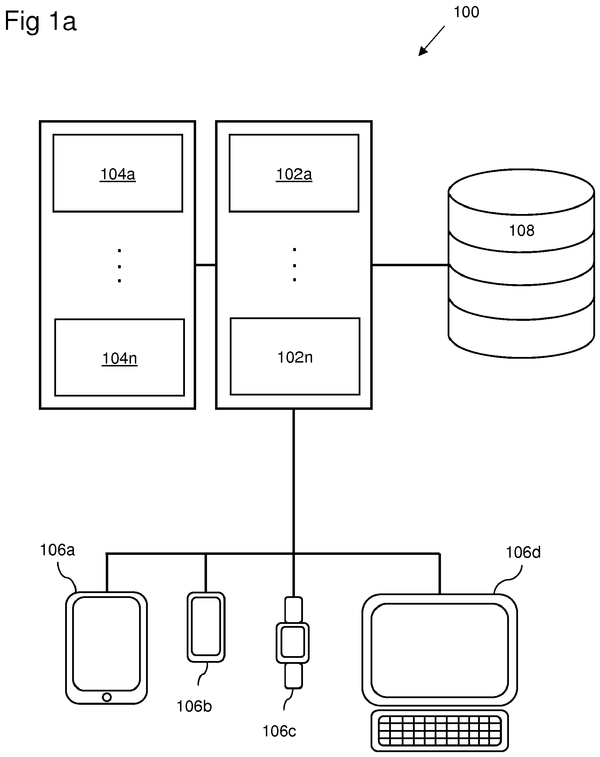

Figure 1a shows a diagram of a system 100 according to an example. The term "system" may refer to any combination of hardware, computer program code, functions, and virtualized resources embodied in a single, or across a plurality of devices. For example, the system 100 may comprise a single device housed in one location, for example, a computer in a hospital, or the system 100 may comprise a plurality of devices housed in the one location, connected over a local area network, for example, a mainframe computer communicatively coupled to at least one other computing device in a hospital. Systems comprising multiple computing devices may be more secure than systems comprising single computing devices as multiple devices must fail for the system to become dysfunctional. It may be more efficient to use a system comprising a plurality of connected computing devices stored remotely from one another rather than having multiple systems.

In other examples, system 100 may comprise a plurality of remote devices. The plurality of remote devices may be connected over a metropolitan area network, a campus area network, or a wide area network, for example, the internet. The system 100 may comprise a plurality of servers and/or mainframe computing devices distributed in hospitals within a country. In other examples, the system 100 may be distributed across multiple countries.

The system 100 comprises at least one processor 102a-n. The at least one processor 102a-n may be a standard central or graphical processing unit (CPU or GPU), or a custom processing unit designed for the purposes described herein. Each of the at least one processors 102a-n may comprise a single processing core, or multiple cores, for example four cores or eight cores. In examples, wherein the system 100 comprises a plurality of processors 102a-n, the processors 102a-n may be embodied in a single device. In other examples the at least one processor 102a-n may comprise multiple processors remotely distributed within the system 100.

The system 100 shown in Figure 1a comprises at least one memory 104a-n. The at least one memory 104a-n may be a non-transitory computer-readable memory, for example a hard-drive, a CD-ROM disc, a USB-drive, a solid-state drive or any other form of magnetic storage device, optical storage device, or flash memory device. The at least one memory 104a-n may be referred to as a storage medium or a non-transitory computer readable storage medium. The at least one memory 104a-n may be maintained locally to the rest of the system 100 or may be accessed re-motely, for example, over the internet. The at least one memory 104a-n may store computer program code suitable for the function described herein. The computer program code may be distributed over multiple memories or may be stored on a single memory.

In an example, the system 100 refers to a system operable to transmit healthcare data to a user device. The system 100 may be operated via a user device, by a user analysing medical information. For example, a doctor, a nurse, a clinical assistant, and others analysing medical information for a patient. Medical information may relate to data, for example, numerical test results from a diagnostic test. Medical information may also relate to qualitative diagnoses and notes relating to a patient or patients.

Figure 1a shows four examples of user devices 106a-d. A user device may be any combination of hardware and computer program code operable by a user and suitable for the function described herein. The user device 106a is a tablet computer, user device 106b is a smart phone, user device 106c is a smart watch, and user device 106d is a personal computer, for example a desktop or laptop PC. It is to be understood that other examples of user devices are also anticipated. User devices 106a-d may comprise any number of volatile or non-volatile memories, processors, and other electronic components. In some examples a user device 106a-d comprises multiple components distributed over a network. A user device 106a-n may comprise any number of outputs, for example a display, a speaker, a tactile feedback system, an LED indicator, a transmitter or any other output. A user device 106a-d may comprise any number of inputs, for example, a microphone, a button, a camera, a receiver, or any number of sensors etc. In some examples the input and output of the user device 106a-d may be considered a user interface, for example, a touch screen or a combination of a screen and a keyboard. A user device 106a-d may be considered part of the system 100 or may not be part of the system 100 but may communicate with the system 100. In some examples a user device 106a-a is local to the system 100 and may be connected to the system 100 over a local area network, for example, a personal computer 106d in a hospital connected to a mainframe computer in the same hospital comprising the system 100. In other examples the user device 106a-d may connect to the system 100 via a wide area network.

The user device 106a-d may be configured for use in analysing medical information. For example, the user device 106a-d may comprise an application for presenting medical information to a user for analysis. In some examples there are multiple user devices 106a-d. The multiple user devices 106a-d may be communicatively coupled to one another. At least one user device 106a-d may be used to control the system 100.

In some examples, a user device 106a-d may be a proprietary device configured to be used in or with the system 100. For example, the user device 106a-d may be a proprietary computing device comprising a combination of firmware, software, and custom applications for providing data and/or other information to a user. For example, the user device 106a-d may comprise applications for displaying data received by and/or transmitted to the system 100 in a predetermined way.

In other examples, the user device 106a-d and the system 100 are comprised in the same device, for example, a desktop computer at a hospital.

In other examples, a user device 106a-d may be a commercially available computing device comprising any number of applications operable to access data at, receive data from, or transmit data to, the system 100. For example, the system 100 may maintain at least one web page, hosted on a remotely accessible server. The user device 106a-d may comprise a web browser operable to access the at least one webpage and thereby facilitate communication with the system 100 and/or display data stored by the system 100 on the user device 106a-d.

The system 100 shown in Figure 1a comprises a database 108 for storing data according to embodiments described herein. The database 108 may be any structured set of data held in a computing device. For example, the database 108 may be structured data stored in the at least one memory 104a-n. In other examples, the database 108 may be stored elsewhere in the system, for example on a separate computing device. The at least one processor 102a-n may be communicatively coupled with the database 108 such that the at least one processor 102a-n may maintain the data-base 108. Maintaining the database 108 may comprise sending data to, receiving data from, or reconfiguring data in the database 108. In examples where the database 108 is stored on physical memory associated with the system 100, the at least one processor 102a-n may be configured to read and/or write data to the physical memory to maintain the database 108. The database 108 may comprise a plurality of databases associated with one another. The database 108 may be embodied by any suitable data structure.

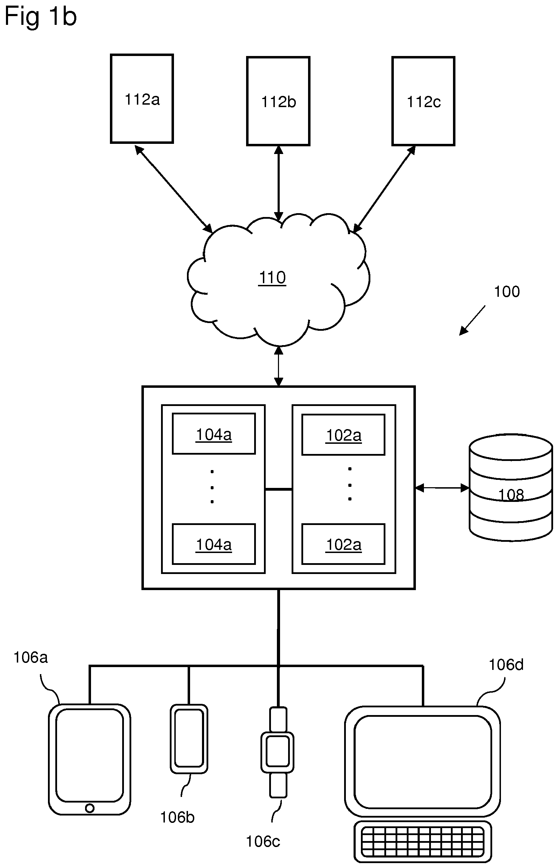

In some examples, the system may be able to communicate with other systems or remote data sources. In an example shown in Figure 1b, the system 100 is connected over a network 110 to at least one remote computing device 112a-c. For example, the system 100 may comprise a plurality of computers and servers located at a hospital, the system 100 may communicate with a computing device 112a, which may alternatively be referred to as a computer system, at another hospital over the network 110 to send and/or receive medical data.

The system 100 may simultaneously be in communication with a computing device 112b representing a medical guideline repository storing at least one medical guideline. A medical guideline repository may be embodied in any device storing at least one medical guideline. In some examples, a medical guideline repository may comprise a remotely accessible database storing at least one guideline, wherein the guideline is stored in a digital format and comprises metadata identifying the at least one guideline. For example, the medical guideline may be stored as a PDF and the metadata may comprise an indication of the name of the medical guideline, a date of publication, an indication of a disease to which the medial guideline relates, a country in which the medical guideline was first published or in which the medical guideline was designed to be applicable to, and any other which may be used to identify a guideline. More examples of identifying features of medical guidelines and their uses will be described later.

The system may also be in communication with a computing device 112c acting as a control device to control the operation of the system 100. For example, a remote computing device such as a personal computer or a server which may be used by an administrator to control the system 100.

Healthcare data as described herein may comprise data relating to: patient records (for example, results from diagnostic tests or therapeutic steps), medical guidelines (for example a directed graph as will be discussed below), statistical data, medical research, scientific articles, or any other medically or clinically relevant information. Healthcare data may be stored in any number of digital formats, the digital format in which the healthcare data is stored may be dependent on the type of healthcare data. For example, raw data relating to diagnostic results may be stored as plain text files, CSV files, or any other suitable file format. Some types of healthcare data may be stored in a human readable format or alternatively may be stored in computer interpretable language. In some examples, the system 100 may transmit healthcare data to a user device 106a-d in a computer interpretable format and the user device 106a-d may process the data and present it to a user in a human readable format.

A medical guideline may define clinical pathways for treating patients with a medical condition. In some examples, a medical guideline is be divided into a series of treatment phases. Examples of treatment phases may include: staging, initial treatment, active surveillance, recurrent treatment, and others. Clinical pathways may be defined by a series of clinical steps, wherein the choice of which clinical step to perform at a given time is dependent on at least a result from at least one previous clinical step. Clinical steps may include, observations, decisions, events, diagnostic tests or therapeutic treatments to be delivered to a patient with a medical condition. Medical guidelines may be printed or published online in a digital format such as PDF. Medical guidelines may contain evidence and/or consensus-based recommendations for medical treatment pathways. Medical guidelines may also contain explanations and/or justifications for the clinical pathway defined within the respective medical guideline.

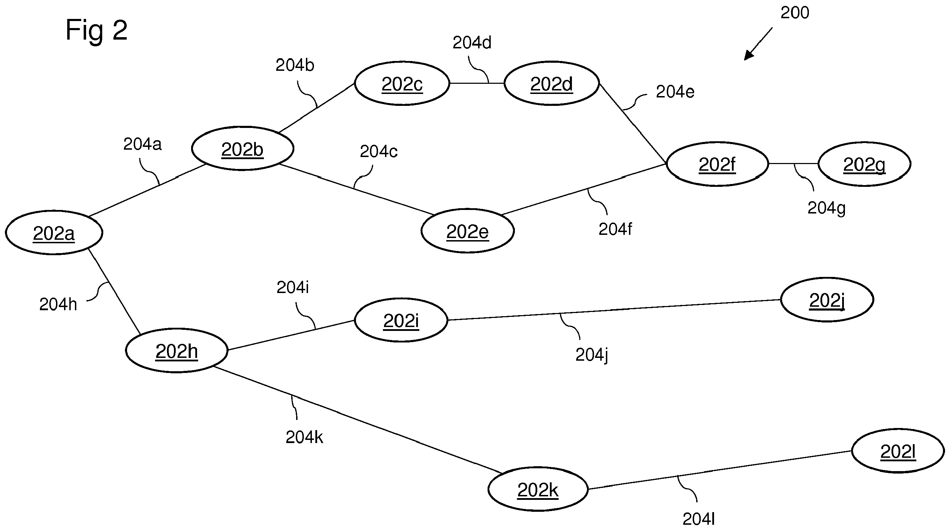

In examples described herein the system 100 maintains in the database 108 a medical guideline represented by at least one directed graph. In some examples a set of directed graphs is used to represent a medical guideline, for example with each directed graph representing a treatment phase within the medical guideline. In the forthcoming discussion reference may be made to a medical guideline being represented by a directed graph. However, it is acknowledged that a medical guideline may represented by a set of directed graphs and reference to a directed graph representing a medical guideline may refer to a directed graph representing at least part of a medical guideline. In an example, a set of directed graphs connect to each other to form a representation of a medical guideline. The system 100 may be preinstalled with machine interpretable representations of the latest medical guidelines. An example of a directed graph can be seen in Figure 2. The directed graph 200 comprises a plurality of elements. The plurality of elements includes a plurality of nodes 202a-l. The nodes 202a-1 may represent clinical steps and in some examples, each of the plurality of nodes 202a-1 represents a clinical step described in the respective at least part of a medical guideline represented by the directed graph 200. The directed graph 200 also comprises a set of directed edges 204a-1. Each node of the plurality of nodes 202a-1 is connected to at least one other node of the plurality of nodes 202a-1 by at least one of the set of directed edges 204a-1. The plurality of nodes 202a-1 and the set of directed edges 204a-1 may be collectively referred to as a elements of the directed graph. The plurality of nodes 202a-1 may define a series of diagnostic tests and medical treatments which are recommended to be performed on a patient with a specific medical condition. The nodes 202a-1 may also define observational points and/or decisions that happen along a treatment pathway. The directed edges 204a-1 may define the direction and/or the order in which the clinical steps are to be performed when treating a patient with a particular medical condition. In some examples, the directed edges define conditions under which a patient is to move from undergoing a particular clinical step represented by a node, for example node 202c, to undergoing a different clinical step represented by a further node, for example 202d. Each of the nodes may represent a conditional parameter value resulting from a said clinical step associated with the node. Alternatively, each of the set of directed edges may represent a conditional parameter value resulting from a said clinical step associated with one of the plurality of nodes connected thereto. In some examples, at least one of the sets of directed edges may specify a user input to be received before traversing from one node to another node. A directed graph may also include a further set of nodes which are not associated with a clinical step.

In an example, a directed graph is maintained in JSON format as at least one list comprising unique identifiers representing nodes 202a-1 and directed edges 204a-1 of a directed graph. The entries in the at least one list may be linked to definitions or references in the respective guideline. In some examples this link comprises other information, for example, a consensus-based weighting. The entries which represent directed edges 204a-1 may also comprise an association or link to nodes 202a-1 connected to the directed edge in the respective directed graph. The entries which represent directed edges may also comprise information on a direction from a first node connected to the directed edge to a second node connected to the directed edge. For example, the entries representing a directed edge 204c may comprise indicating that the directed edge 204c is connected to nodes 202b and 202e and that the direction along the directed edge 204c is from 202b to 202e. The directed graphs may comprise two layers of elements allowing modifications to be made at a clinic and/or clinical site without losing the information relating to the original directed graph.

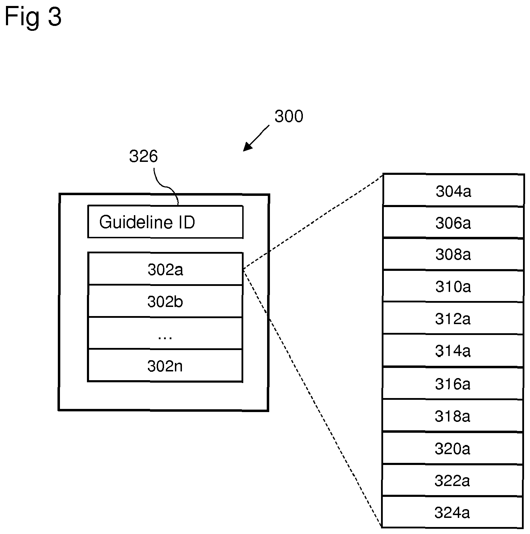

Further information relating to a directed graph may be maintained in an event model. Figure 3 illustrates an example of an event model for a directed graph. The event model 300 comprises a list of entries 302a-n, representing events or steps, linked to respective nodes in the directed graph. The entries 302a-n may comprise any of: a unique identifier 304an, encoding in a medical coding system 306a-n, a label 308an, a type of step 310a-n(e.g. biopsy), a required patient attribute input 312a-n, a required patient attribute output 314a-n, annotations relating to: impact of event 316a-n, effectiveness of event 318a-n, cost of event 320a-n, duration of event 322a-n, invasiveness of event 324a-n, or any other relevant information. Thereby allowing important information contained in a medical guideline to be stored in an efficient manner allowing the use of medical guidelines as described herein. The event model 300 comprises an identifier 326 relating the event model to a respective medical guideline. In Figure 3 only the data for the entry 302a is shown for clarity.

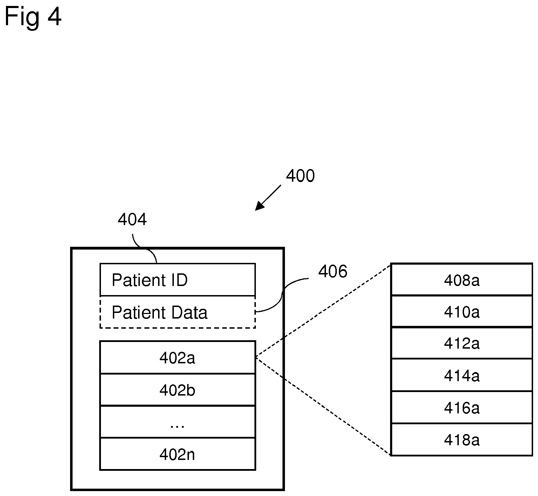

In certain examples described herein the system 100 maintains, in the database 108, a plurality of patient models each comprising healthcare data associated with a respective patient. For example, the patient models may comprise any of: data generated during clinical procedures such as diagnostic and/or therapeutic steps performed on a patient; reasons for performing a clinical procedure on a patient, for example, especially where the choice of clinical procedure deviates from a medical guideline; general data relating to a patient such as age, gender, height, body mass index; known conditions, risk factors, a patient identifier; genetic information relating to the patient; or any other information classifying the patient. In some examples, the patient models may each be stored as a list comprising a plurality of patient entries. Each patient entry may comprise data relating to a patient attribute. Figure 4 shows an example of a patient model 400 comprising a list of patient entries 402a-n, a patient identifier 404, and in some cases other patient data 406. The patient entries 402a-n may comprise any of: a unique identifier 408a-n, an encoded identifier 410a-n such as an identifier in a medical encoding system, a natural language label 412a-n, a type of clinical step 414a-n (e.g. biopsy, scan, a physical assessment, etc.) a measurement unit 416a-n, and a patient attribute value 418a-n (for example, the result from a test). The patient entries may comprise an association between the encoded identifier 410a-n and the patient attribute value 418an also called an attribute value. In the example shown in Figure 4 only the data in patient entry 402a is shown for clarity. The encoded identifier may identify the clinical step to which the patient attribute value of the respective patient entry relates, wherein each clinical step is associated with a respective encoded identifier.

In some examples, a plurality of patient models relating to patients are stored and/or maintained in a central database 108 or computing device. The patient models may be stored in the same database as the data representing directed graphs or may be stored in a separate database. The patient models may be accessible through the system 100 over a network connection. In other examples, patient models may be stored locally with the clinical centre, for example a doctor's surgery or a hospital, at which the patient has been treated in the past or is currently receiving treatment. Patient models stored at one hospital in the system 100 may be accessed at remote locations through the system 100, for example, over the network 110.

In some examples, the system 100 updates patient models by accessing remote computing devices 112a-c over the network 110. For example, the system 100 may access medical testing equipment storing data relating to a patient, over the network 110 to update a respective patient model. The system 100 may access servers or other computing devices in hospitals which store medical information relating to patients. In some examples, the system 100 may continuously and/or regularly collect data about patients from various hospital information systems. Data may be collected at predetermined intervals for example, every hour, every day, etc. The size of the interval may be dependent on the size of the system 100 or the clinical centres. In some examples, data collection may be triggered by other events and/or messages occurring in the system 100. The retrieval of the data about the patients may be based on existing standards, for example, HL7, DICOM, FHIR. In other examples the retrieval of the data about the patients may be performed by using proprietary information about information storages available in a hospital. In some examples the retrieval of patient data is performed by receiving or accessing data, from external sources, comprising encoded identifiers such as SNOMED CT, LOINC, or Siemens internal coding system. In other examples, the system 100 may use natural language processing techniques to extract information from electronically stored notes and files relating to a patient. In some examples, a combination of both techniques may be used. The patient model may be represented in the system 100 as a set of resources conformant with FHIR standard and stored in an FHIR server.

The following description of embodiments of the inventions will be described with reference to the example system 100 of Figure 1. However, the following embodiments may be implemented in systems different to that of Figure 1.

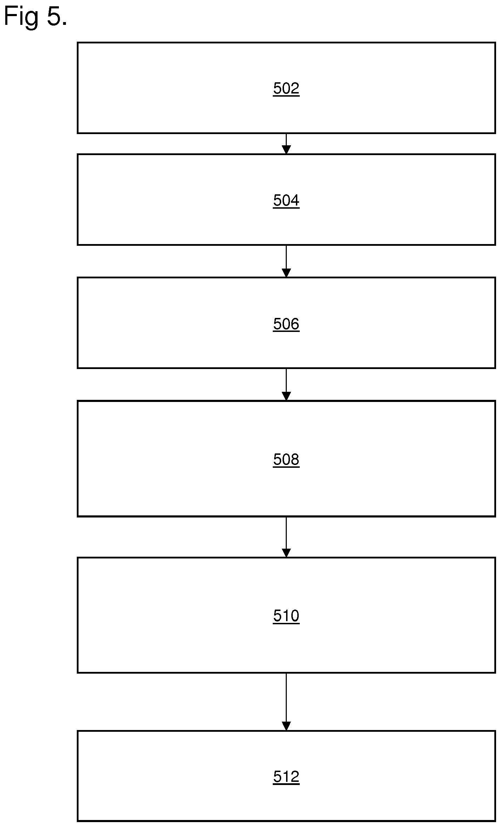

In an embodiment the at least one memory 104a-n includes computer program code and the at least one memory 104a-n and computer program code are configured to, with the at least one processor 102a-n, cause the system 100 to perform the steps indicated by each of the blocks of the flow chart in Figure 5. Wherein, at block 502, the system 100 maintains, in a first database 108, data representing a first directed graph representing at least part of a medical guideline, the first directed graph comprising a plurality of elements representing clinical step. In some implementations the plurality of elements include a plurality of nodes each representing a clinical step and a set of directed edges, each node of the plurality of nodes being connected to at least one further node by one of the set of directed edges, the first directed graph comprising a primary node and terminating in at least one end node. At block 504, the system 100 maintains, in a second database 108, a plurality of patient models each comprising healthcare data associated with a respective patient. The first and second databases may be the same or separate databases.

At block 506, the system 100 selects at least one element, such as a node 202a-l, a directed edge 204a-l, or a combination of the two, from the plurality of elements by processing the plurality of patient models and the data representing the directed graph to identify at least one element at which treatment of a subset of patients has deviated from the at least part of a medical guideline. This may involve comparing a required patient attribute inputs 312a-n or a required patient attribute outputs 314a-n for each node 202a-1 and/or directed edge 204a-1 with respective patient attribute values, from each patient model. In some examples, the element which is selected may be an element at which the number of patients whose treatment has deviated from the at least part of a medical guideline exceeds a predetermined number.

Alternatively, selecting the at least one element may comprise processing the plurality of patient models and the data representing the first directed graph to identify at least one element at which the subset of patients whose treatment has deviated from the at least part of the medical guideline exceeds a predetermined proportion of the patients associated with the plurality of patient models. For example, there may be a selected ratio or percentage. Wherein if the number of patients whose treatment deviates at a node and/or directed edge exceeds a given percentage of the total number of patients, then the node and/or directed edge may be selected.

At block 508, the system 100 identifies, based on a combination of the at least one selected element and the plurality of patient models, a first patient cohort whose treatment has deviated from the at least part of a medical guideline at the at least one selected element and a second patient cohort whose treatment has conformed to the at least part of a medical guideline at the at least one selected element. In some cases, the system 100 may override the selection of the at least one element based on a user input.

The treatment of patients represented by the plurality of patient models may deviate at a selected node in a plurality of ways. Nodes in the first directed graph may be either decision nodes or outcome nodes. A decision node is a node at which a decision to perform a specific clinical step, such as a treatment procedure, a medical test, or another active clinical step, is to be performed. An outcome node is a node corresponding to an analysis of a result of an active clinical step. For example, a decision node may represent a decision to perform a blood test of a patient, and an associated outcome node may be a node representing an analysis of the blood test wherein variables in the results of the blood test have a desired range. A patient's treatment may deviate at the decision node if a medical practitioner decides not to perform the recommended blood test. A patient's treatment may deviate at the outcome node if the results of their blood test are not within the desired range associated with the outcome node.

A described above, the selected node may be associated with at least one conditional parameter value, the patient models each comprise a plurality of patient attribute values corresponding to respective clinical steps, and the first and second patient cohorts are identified based on a comparison of the at least one conditional parameter value with respective patient attribute values from the plurality of patient models. In this way, patients whose attribute values associated with a clinical step do not conform with the expected values at that clinical step can be identified as belonging to the first patient cohort.

A patient deviates from a decision node where the treatment of the patient does not conform to the decision represented by the node. In an example of a guideline for the therapy of a certain type of cancer. A decision node in an associated directed graph representing at least part of this guideline may represent a possible choice of therapy depending on the subtype of cancer and/or certain laboratory values. A possible output at the decision node may be an option recommending radiotherapy followed by chemotherapy. In many cases this recommendation may be adhered to and it can be determined that this is the case by identifying data associated with the recommended steps in the respective patient models (e.g. test results from radiotherapy and chemotherapy stored in the patient models). Alternatively, an indicator that the recommended steps were performed may be input to the system 100 by a medical practitioner and stored with the patient model.

However, for some patients, chemotherapy may not be the best form of treatment. For example, elderly patients may not survive the side effects of chemotherapy. A physician may instead choose to perform only radiotherapy and not chemotherapy, because the physician is aware that this will lead to a better outcome for the patient, such as prolonged life and/or a better quality of life for the patient. In this case the first and second patient cohorts are identified based on an availability of healthcare data associated with the respective patient models corresponding to the selected node. The patient entries stored in the patient models may each be associated with relative order and/or a date on which the patient entries were generated. In this way it is possible for the system 100 to determine whether the patient adhered to the medical guideline based on a correspondence between the order in which clinical steps are recommended by the guideline and the patient entries in the respective patient models.

Similarly, where directed edges are associated with conditional parameter values, the treatment of patient deviates at a directed edge if the conditional parameter values associated with the directed edge are not satisfied by corresponding patient attribute values. The treatment of a patient may also deviate at the directed edge if the treatment of the patient deviates from clinical steps represented by nodes connected to the directed edge. In some implementations, directed edges represent clinical steps.

Associating each patient entry with a respective date and/or relative order may allow patient models to store medical information relating to an entire patient history without interfering with an analysis of a current treatment phase. In this case the patient model may comprise a patient entry relating to a clinical step which was to be performed. However, the patient entry may relate to a prior performance of the clinical step which occurred in a previous treatment phase or at a much earlier date. For example, a patient may be treated for a specific cancer and so undergoes both radiotherapy and chemotherapy. After a period of remission, the cancer may recur, or a new cancer may develop in the patient. The patient may be treated again but in this case the patient may not adhere to the guideline at the decision node indicating that radiotherapy and chemotherapy are to be performed, e.g. because the patient has become much older and this is no longer a recommended option. In this case, it is important to distinguish between patient entries in the patient model which relate to the first occurrence of the cancer and the reoccurrence of the cancer. In this respect, the patient entries may each be associated with a date such that a timeline of the patient history can be accurately mapped to the directed graph.

At block 510, the system 100 processes the plurality of patient models representing the first and second patient cohorts to determine at least one patient cohort characteristic distinguishing the first patient cohort from the second patient cohort. At block 512, the system 100 generates a second directed graph dependent at least on the at least one identified patient cohort characteristic. At block 514, the system 100 transmits data representing the second directed graph for receipt by the user device 106a-d.

In some cases, the reasons for non-adherence to medical guidelines may, for example, be due to as of yet unidentified shortcomings in the medical guidelines, divergence of practice in different medical care facilities, availability of equipment, and other factors which may impact the ability to, and/or the effectiveness of, adhering to a medical guideline. By identifying a patient cohort characteristic which distinguishes the first cohort of patients from the second cohort of patients it may be possible to identify the causes for non-adherence to the medical guidelines at the at least one selected element. Many different factors may be considered as patient cohort characteristics including but not limited to, age, height, weight, sex, body mass index, genetic mutations, pre-existing conditions, associated medical practitioners, and locations.

As described above, a patient diverges at an outcome node if a patient attribute value corresponding to the respective clinical step is divergent from an expected value such as a conditional parameter value. Divergence from the expected values may indicate a poor outcome for the patient. By identifying a patient cohort characteristic of patients who have had a poor outcome from following the medical guideline it may be possible to make treatment for future patients who share the identified patient cohort characteristic more effective. To this end, the second directed graph may comprise an indication that the clinical step represented by the at least one selected element is not recommended for patients associated with the at least one identified cohort characteristic.

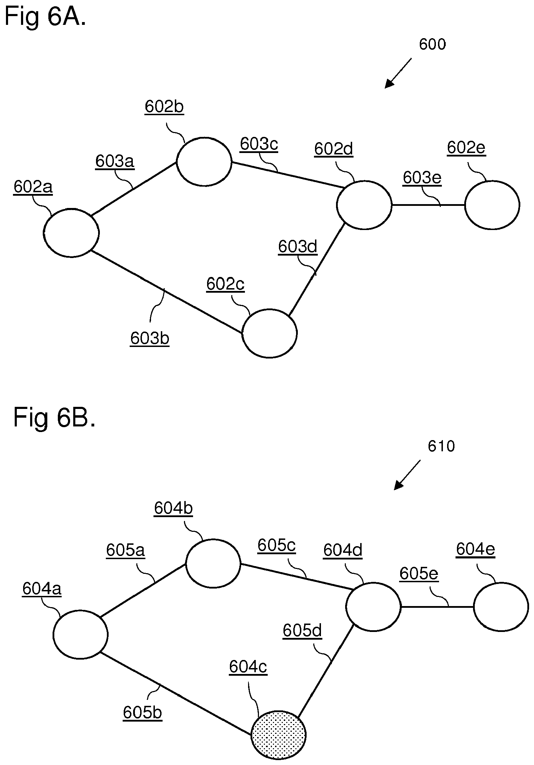

Figure 6A shows a first directed graph 600 representing a part of a medical guideline for treating specific type of cancer. The directed graph 600 comprises a plurality of nodes 602a-e and set of directed edges 603a-e. The node 602c represents the decision to perform radiotherapy and chemotherapy. In an example, the node 602c is selected and it may be determined that a first patient cohort whose treatment deviated from the part of a medical guideline at the node 602c are distinguished from a second patient cohort because the patients in the first cohort share the characteristics of being males over the age of 75. Figure 6B shows a second directed graph 610 which is dependent on at least the identified characteristics. The second directed graph 610 comprises a plurality of nodes 604a-d corresponding to respective nodes in the directed graph 600, wherein reference numerals with a common alphabetic suffix indicate nodes representing the same clinical step. E.g. node 602a represents the same clinical step as node 604a, and node 602b represents the same clinical step as node 604b, etc. The second directed graph 610 also comprises a set of directed edges 605a-e corresponding to respective directed edges in directed graph 600, wherein reference numerals with a common alphabetic suffix indicate that the edges correspond to each other. The directed graph 610 also comprises an indication that the clinical step represented by the selected node 602c, 604c is not recommended for patients associated with the at least one identified characteristic, which in this case includes being male and over 75 years of age. The indication, in the example of Figure 6B, is implemented as a modification of the appearance of the node 604c. The data representing the second directed graph may also cause a user device 106a-d to generate an alert associated with the selected node 604c. For example, while mapping a patient model to the directed graph 610 during treatment of a patient, if the patient model is mapped to a node before or at the selected node 604c an alert may be displayed in conjunction with the selected node 604c on the user device 106a-d indicating that the clinical step represented by the selected node 604c is not recommended for patients who are male and over the age of 75 (i.e. who share the identified at least one patient cohort characteristic of the first patient cohort).

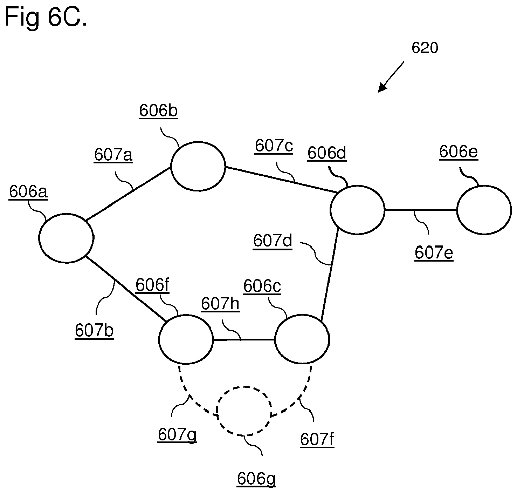

Figure 6C shows a further example of a second directed graph 620 which is dependent on at least the identified characteristic. The comprising a plurality of nodes 606a-e corresponding to respective nodes in directed graph 600 and 610 wherein reference numerals with a common alphabetic suffix indicate nodes representing the same clinical step. E.g. node 602a represents the same clinical step as node 606a, and node 602b represents the same clinical step as node 606b, etc. The second directed graph 620 also comprises a set of directed edges 607a-h corresponding to respective directed edges in directed graph 600, wherein reference numerals with a common alphabetic suffix indicate that the edges correspond to each other. The directed graph 620 also comprises a further node 606f connected to the selected node 606c via a respective directed edge 607h. The further node 606f comprises the indication that the clinical step represented by the selected node 606c is not recommended for patients associated with the at least one identified patient cohort characteristic. In this way, a physician or another medical practitioner may be notified of the potential deviation before the patient is at the node 606c where the deviation has been identified as likely to occur for patients having the at least one identified patient cohort characteristic.

In some examples, the directed graph 620 comprises more than one further node 606f, for example the extra node 606g shown in broken lines in Figure 6C. In this case, when mapping a patient model to the directed graph 620 a potential deviation may be identified at node 606f and then a further clinical step of checking whether the patient model being analysed represents a patient associated with the at least one identified patient cohort characteristic may be performed at node 606g.

In other examples, the second directed graph 620 comprises one further directed edge connected at a first end to the further node, the further directed edge being indicative of a deviation from the at least part of a medical guideline for treatment of the first patient cohort. For example, a directed edge may indicate that the treatment of the first patient cohort should deviate from the guideline before the clinical step represented by the selected node. The further directed edge indicates that a physician should decide how to treat the patient, for example using their knowledge to determine a preferred method of treatment.

The system 100 may, based on a user input indicative of a decision in respect of patients associated with the at least one identified patient cohort characteristic, modify the second directed graph. In other words, the system 100 may automatically modify the second directed graph to include changes to a treatment phase for patients in the first patient cohort. The system 100 can detect where users make treatment decisions, which deviate from the guideline, for the treatment of patients in the first cohort and modify the second directed graph to reflect this.

A further patient model may be analysed using the second directed graph 610 or 620, the further patient model being stored in a third database and comprising healthcare information associated with a patient who is associated with at least one identified characteristic of the first patient cohort. The system 100 may determine, based on a combination of the second directed graph and the further patient model, a status of the at least one further node and/or a status of a directed edge connected thereto. In this case, the status of a node or directed edge connected to the node refers to the availability and/or characteristics of data stored in a patient model corresponding to the clinical step represented by the respective node. For example, the current position of a patient in relation to a medical guideline can be determined based on a comparison of patient entries stored in the patient model and conditional parameter values corresponding to nodes and/or directed edges.

The system 100 may then, dependent on the status of the at least one further node and/or the status of the directed edge connected thereto, transmit data indicative of the status of the at least one further node and/or the status of the directed edge connected thereto for receipt by the user device 106a-d. This data is then used for determining whether a clinical step, represented by the selected node, is not recommended for the patient. In this way, when using the second directed graph to inform treatment of a patient, relevant information and/or prompts may be generated informing a physician regarding an updated of the at least one medical guideline based on the identified patient cohort characteristic. The system 100 may in fact prompt the physician to check some medical information relating to the patient to determine if a particular clinical step should be performed.

Once a medical practitioner is made aware that a clinical step in the medical guideline is not recommended for this current patient, they may be prompted to update the second directed graph with the clinical steps which they do perform. To this end, the system 100 may, dependent on the status of the further node, transmit data indicative of a request for input from a user of the user device 106a-d for receipt by the user device 106a-d. The system 100 may then receive, from the user device 106a-d, data indicative of further clinical steps to be represented by a further plurality of nodes and modify the second directed graph based on the received data indicative of the further clinical steps.

Processing the plurality of patient models representing the first and second patient cohorts to determine the at least one patient cohort characteristic may use a number of suitable statistical and machine learning techniques. For example, the processing may involve at least one of principal component analysis, random forest regression, and regularized regression.

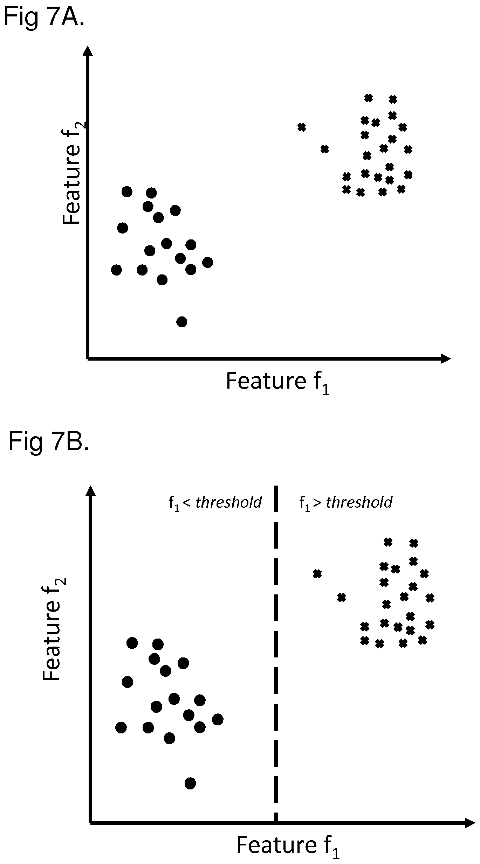

Figures 7A to 7C illustrate graphically how statistical methods may be used to identify distinguishing cohort characteristics. Let us consider a plurality of patient models each comprising N characteristics, or features, which describe a patient (f₁, f₂, ..., f_{N}). These features can include demographic (gender, age, etc.), clinical findings and observations, previous exams, current medication, allergies, smoking history, etc.

Figure 7A shows a plot of patient models based on two such features (f₁, f₂) wherein a first subgroup of patients is shown using circles and a second subgroup are shown with crosses. Although a plot of patient models is shown using two such features it is to be understood that the differences between patients may be analysed based on a greater number of variables. The statistical methods may involve analysing a feature space to identify features that allow one to distinguish the two groups. In the example shown in Figure 7A and 7B, it is possible to distinguish between the groups based on a single feature, in this case f₁. Automatic analysis can be performed to identify the threshold to separating the two subgroups.

A Classification and Regression Tree model (CART) can be trained to classify patient models into one of the two classes, and thereby performs feature selection internally. The selected features, which are generated as a by-product of the CART training, can be used as the discriminating features and form the at least one patient cohort characteristic. Other classification techniques may also be used as discussed above, including linear discriminant analysis (LDA), random forests, and support vector machines (SVMs).

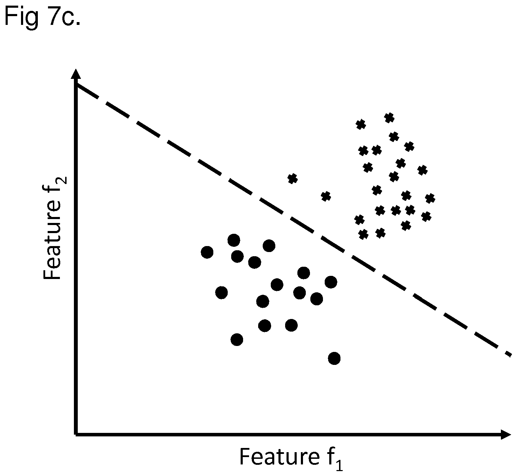

In some examples, such as that shown in Figure 7C, the groups cannot be distinguished by a single feature. Instead a linear or non-linear combination of features may be used to distinguish between the two subgroups. In this case, modified version of the techniques described above may be used to generate hyperplanes for any number of features N given the two subgroups of patient models. In the example of Figure 7c, the hyperplane is implemented as a classification line.

Where multiple directed graphs are available which representing the same at least part of a guideline, for example, where one directed graph comprises a modification to the guideline which is either manually or automatically generated, the system 100 may test the suitability of each directed graph and select the most favourable one.

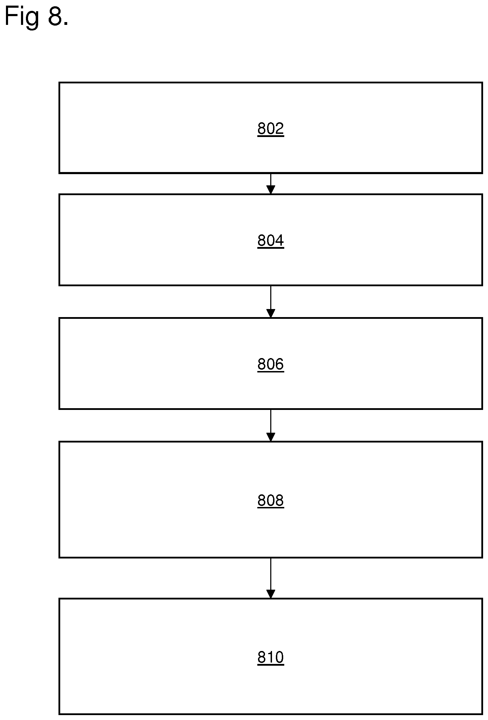

In an embodiment the at least one memory 104a-n includes computer program code and the at least one memory 104a-n and computer program code are configured to, with the at least one processor 102a-n, cause the system 100 to perform the steps indicated by each of the blocks of the flow chart in Figure 8.

At block 802, the system 100 maintains, in a first database 108, data representing a first directed graph representing at least part of a medical guideline and a second directed graph representing the at least part of a medical guideline and a modification to the at least part of a medical guideline.

The modification to the at least part of the medical guideline may include a deviation in practice from the recommended clinical steps indicated in the guideline. For example, as described above, certain clinical steps may be avoided when treating certain types of patients, a second directed graph may represent such a deviation in treatment. The second directed graph representing the modification may be manually created for example, based on a hypothesis of a medical practitioner, or may be automatically generated based on patient histories as represented by patient models.

Each of the first and second directed graphs comprise a respective plurality of elements representing a clinical step. The plurality of elements may comprise a plurality of nodes and a set of directed edges, wherein the plurality of nodes represent respective clinical steps.

At block 804, the system 100 maintains in a second database 108, a plurality of patient models each comprising healthcare data associated with a respective patient. The first and second databases may be the same database or separate databases.

At block 806, the system 100 identifies a first set of patient models and a second set of patient models. The first set of patient models represent patients that have been treated based on the at least part of a medical guideline as represented by the first directed graph. The second set of patient models represent patients that have been treated based on the at least part of a medical guideline as represented by the second directed graph. Where the modification is dependent on a patient cohort characteristic, the first and second set of patient models may represent patients who are associated with the patient cohort characteristic. In other examples, the patient models are a random, or near random, sample of patient models stored in the second database. The modification to the at least part of a medical guideline may be represented in the second directed graph by at least one of at least one node, and at least one directed edge, as discussed previously in relation to Figures 6A to 6C. Similarly to the examples described above, the modification to the at least part of the medical guideline is associated with a patient cohort characteristic and the first and second sets of patient models may be identified based on the patient cohort characteristic.

At block 808, the system 100 determines, based on a comparison of the first set of patient models with the second set of patient models, which of the first and second directed graphs is a preferred directed graph. At block 810, the system 100, responsive to the determination, transmits data representing the preferred directed graph for receipt by the user device 106a-d.

A preferred directed graph is generally a directed graph which, when implemented in the treatment of patients by a physician, leads to better outcomes for those patients. A better outcome for a patient is an outcome which provides any of improved well-being, better quality of life, longer life expectancy, fewer post treatment complications, and in some cases reduced likelihood of recurrence of ailment.

The system 100 may determine which of the first or second directed graphs is a preferred directed graph based on the plurality of patient models. In this embodiment, the patient models each comprise at least one patient outcome measure, for example in a respective patient entry or entries. The system 100 may compare a first plurality of patient outcome measures of the first set of patient models with a second plurality of patient outcome measures of the second set of patient models.

The outcome measures in the patient models may be generated automatically, for example, based on data generated from apparatus involved in treatment phases, including test results. In other examples, the outcome measures may be gathered and manually entered based on an input from a user. In this case, a physician who is treating a patient may, during the treatment of the patient and/or during a follow up process after treatment, generate data representing outcome measures. These may be entered to a user device 106a-d and transmitted for storing in the respective patient models.

The first set of patient models may be identified by the system 100 based on a comparison of the plurality of patient models and the first directed graph. The comparison of the patient models and the first directed graph may involve determining a status of each of the elements of the first directed graph for each patient model. Similarly, the second set of patient models may be identified based on a comparison of the plurality of patient models with the second directed graph. Alternatively, the second set of patient models may be determined based on a process of elimination following the identification of the first set of a patient models.

To determine the status of an element, the system 100 may maintain a first association, between at least one of the patient entries and an identifier from a plurality of identifiers, and a second association between the element and the identifier. An attribute value associated with the element is selected based on the first and second associations. The system 100 then determines, based on a comparison of attribute value associated with the said element to a conditional parameter value associated with the said element, whether the conditional parameter value associated with the said element is satisfied.

The determination of which directed graph is a preferred directed graph may also be sensitive to how well patients adhere to each directed graph. For example, patients who are treated according to the second directed graph may be more likely to deviate than patients who are treated according to the second directed graph, which may be an indicator of an unsuitability of the second directed graph. To do this, the system 100 may determine a first measure, indicative of an average conformity of the first set of patient models to the first directed graph, and a second measure which is indicative of an average conformity of the second set of patient models to the second directed graph. The comparison of the first plurality of patient outcome measures and the second plurality of patient outcome measures may be performed using the first and second measures of average conformity. The first and second measures of conformity are dependent on average statuses for (i) the first set of patient models and the plurality of elements of the first directed graph and (ii) for the second set of patient models and the plurality of elements of the second directed graph respectively.

User input may be used to influence the selection of the first or second directed graphs as the preferred directed graph. To this end, the result of the comparison of the first and second patient models, that is the comparison of the respective outcome measures, may be transmitted for receipt by the user device 106a-d. The system 100 may then receive data indicative of a decision in respect of the result of the comparison from the user device 106a-d and select, based on the received data, one of the first or second directed graphs.

The system 100 may also provide supplementary data to the user device 106a-d along with the data representing the preferred directed graph. The system 100 may transmit data corresponding to the first plurality of outcome measures for receipt by the user device 106a-d if the first directed graph is preferred or the second plurality of outcome measures for receipt by the user device 106a-d if the second directed graph is preferred.

The above examples are given for illustrative purposes. The systems described above may be used in the staging, and management of any patient having a disease for which at least one medical guideline is available.

### Numbered Clauses

The following numbered clauses describe various embodiments of the present invention.
1. A system operable to transmit healthcare data to a user device, the user device being configured for use in analysing medical information, the system comprising at least one processor and at least one memory including computer program code, the at least one memory and computer program code configured to, with the at least one processor, cause the system to:
   maintain, in a first database, data representing a first directed graph representing at least part of a medical guideline, the first directed graph comprising a plurality of elements representing a clinical step;
   maintain, in a second database, a plurality of patient models each comprising healthcare data associated with a respective patient;
   select at least one element from the plurality of elements by processing the plurality of patient models and the data representing the first directed graph to identify at least one element at which treatment of a subset of patients has deviated from the at least part of a medical guideline;
   identify, based on a combination of the at least one selected element and the plurality of patient models, a first patient cohort whose treatment has deviated from the at least part of a medical guideline at the at least one selected element and a second patient cohort whose treatment has conformed to the at least part of a medical guideline at the at least one selected element;
   process the plurality of patient models representing the first and second patient cohorts to identify at least one patient cohort characteristic distinguishing the first patient cohort from the second patient cohort;
   generate a second directed graph dependent at least on the at least one identified patient cohort characteristic; and
   transmit data representing the second directed graph for receipt by the user device.
2. A system according to clause 1, wherein selecting the at least one element comprises processing the plurality of patient models and the data representing the first directed graph to identify at least one element at which the subset of patients whose treatment has deviated from the at least part of the medical guideline exceeds a predetermined proportion of the patients associated with the plurality of patient models.
3. A system according to clause 1 or clause 2, wherein the at least one patient cohort characteristic comprises at least one of:
   an age;
   a height;
   a weight;
   a sex;
   a body mass index;
   a genetic mutation;
   an associated medical practitioner; and
   a location.
4. A system according to any preceding clause, wherein the memory and computer program code are configured to, with the at least one processor, cause the system to override the selection of the at least one element based on a user input.
5. A system according to any preceding clause, wherein the at least one selected element is associated with at least one conditional parameter value, the patient models each comprise a plurality of patient attribute values corresponding to respective clinical steps, and the first and second patient cohorts are identified based on a comparison of the at least one conditional parameter value with respective patient attribute values from the plurality of patient models.
6. A system according to any of clauses 1 to 4, wherein the first and second patient cohorts are identified based on an availability of healthcare data in the respective patient models corresponding to the at least one selected element.
7. A system according to any preceding clause, wherein processing the plurality of patient models representing the first and second patient cohorts to determine at least one patient cohort characteristic distinguishing the first patient cohort from the second patient cohort comprises processing the plurality of patient models using at least one of:
   principal component analysis;
   random forest regression; and
   regularized regression.
8. A system according to any preceding clause, wherein the second directed graph comprises an indication that the clinical step represented by the at least one selected element is not recommended for patients associated with the at least one identified patient cohort characteristic.
9. A system according to clause 8, wherein the second directed graph comprises:
   a plurality of nodes;
   a set of directed edges; and
   at least one further node connected to the selected element,
   wherein the at least one further node comprises the indication that a clinical step represented by the selected element is not recommended for patients associated with the at least one identified patient cohort characteristic.
10. A system according to clause 9, wherein the second directed graph comprises at least one further directed edge connected at a first end to the further node, the further directed edge being indicative of a deviation from the at least part of a medical guideline for the first patient cohort.
11. A system according to any preceding clause, wherein the at least one memory and computer program code are configured to, with the at least one processor, cause the system to:
   based on a user input indicative of a decision in respect of patients associated with the at least one identified patient cohort characteristic, modify the second directed graph.
12. A system according to clause 9 or clause 10, wherein the at least one memory and computer program code are configured to, with the at least one processor, cause the system to:
   maintain, in a third database, a further patient model comprising healthcare data associated with a patient, the patient being associated with the at least one identified patient cohort characteristic;
   determine, based on a combination of the second directed graph and the further patient model, a status of the at least one further node and/or the status of a directed edge connected thereto; and
   dependent on the status of the at least one further node and/or the status of the directed edge connected thereto, transmit data indicative of the status of the at least one further node and/or the status of the directed edge connected thereto for receipt by the user device, the data indicative of the status of the at least one further node and/or directed edge connected thereto being for use in determining whether a clinical step represented by the selected node is not recommended for the patient.
13. A system according to clause 12, wherein the at least one memory and computer program code are configured to, with the at least one processor, cause the system to:
   dependent on the status of the further node and/or the status of the directed edge connected thereto, transmit data indicative of a request for input from a user of the user device for receipt by the user device;
   receive, from the user device, data indicative of further clinical steps to be represented by a further plurality of nodes; and
   modify the second directed graph based on the received data indicative of the further clinical steps.
14. A computer program comprising a set of instructions, which, when executed by a computerised device, cause the computerised device to perform a method of transmitting healthcare data to a user device, the user device being configured for use in analysing medical information, the method comprising:
   maintaining, in a first database, data representing a first directed graph representing at least part of a medical guideline, the first directed graph comprising a plurality of elements representing a clinical step;
   maintaining, in a second database, a plurality of patient models each comprising healthcare data associated with a respective patient;
   selecting at least one element from the plurality of elements by processing the plurality of patient models and the data representing the first directed graph to identify at least one element at which treatment of a subset of patients has deviated from the at least part of a medical guideline;
   identifying, based on a combination of the at least one selected element and the plurality of patient models, a first patient cohort whose treatment has deviated from the at least part of a medical guideline at the at least one selected element and a second patient cohort whose treatment has conformed to the at least part of a medical guideline at the at least one selected element;
   processing the plurality of patient models representing the first and second patient cohorts to identify at least one patient cohort characteristic distinguishing the first patient cohort from the second patient cohort;
   generating a second directed graph dependent at least on the at least one identified patient cohort characteristic; and
   transmitting data representing the second directed graph for receipt by the user device.
15. A method of transmitting healthcare data to a user device, the user device being configured for use in analysing medical information, the method comprising:
   maintain, in a first database, data representing a first directed graph representing at least part of a medical guideline, the first directed graph comprising a plurality of elements representing a clinical step;
   maintaining, in a second database, a plurality of patient models each comprising healthcare data associated with a respective patient;
   selecting at least one element from the plurality of elements by processing the plurality of patient models and the data representing the first directed graph to identify at least one element at which treatment of a subset of patients has deviated from the at least part of a medical guideline;
   identifying, based on a combination of the at least one selected element and the plurality of patient models, a first patient cohort whose treatment has deviated from the at least part of a medical guideline at the at least one selected element and a second patient cohort whose treatment has conformed to the at least part of a medical guideline at the at least one selected element;
   processing the plurality of patient models representing the first and second patient cohorts to identify at least one patient cohort characteristic distinguishing the first patient cohort from the second patient cohort;
   generating a second directed graph dependent at least on the at least one identified patient cohort characteristic; and
   transmitting data representing the second directed graph for receipt by the user device.
16. A system operable to transmit healthcare data to a user device, the user device being configured for use in analysing medical information, the system comprising at least one processor and at least one memory including computer program code, the at least one memory and computer program code configured to, with the at least one processor, cause the system to:
   maintain, in a first database, data representing a first directed graph representing at least part of a medical guideline and a second directed graph representing the at least part of a medical guideline and a modification to the at least part of a medical guideline, each directed graph comprising a respective plurality of elements representing a clinical step;
   maintain, in a second database, a plurality of patient models each comprising healthcare data associated with a respective patient;
   identify a first set of the patient models representing patients that have been treated based on the at least part of a medical guideline as represented by the first directed graph and a second set of the patient models representing patients that have been treated based on the at least part of a medical guideline as represented by the second directed graph;
   determine, based on a comparison of the first set of patient models with the second set of the patient models, which of the first and second directed graphs is a preferred directed graph; and
   responsive to the determination, transmit data representing the preferred directed graph for receipt by the user device.
17. A system according to clause 16, wherein the modification to the at least part of a medical guideline is represented by at least one of:
   at least one node in the second directed graph; and
   at least one directed edge in the second directed graph.
18. A system according to clause 16 or clause 17, wherein the plurality of patient models each comprise a plurality of patient entries, at least one of the patient entries including at least one patient outcome measure, and determining which of the first and second directed graphs is a preferred directed graph comprises comparing a first plurality of patient outcome measures of the first set of patient models with a second plurality of patient outcome measures of the second set of patient models.
19. A system according to any one of clauses 16 to 18, wherein the modification of the at least part of a medical guideline is associated with a patient cohort characteristic and the first and second sets of patient models are identified based on the patient cohort characteristic.
20. A system according to any one of clauses 16 to 19, wherein the at least one memory and computer program code are configured to, with the at least one processor, cause the system to identify the first set of patient models based on a comparison of the plurality of patient models and the first directed graph, wherein comparing the plurality of patient models with the first directed graph comprises, for each patient model, determining a status of at least one of the plurality of elements of the first directed graph.
21. A system according to any one of clauses 16 to 20, wherein the at least one memory and computer program code are configured to, with the at least one processor, cause the system to identify the second set of patient models based on a comparison of the plurality of patient models and the second directed graph, wherein comparing the plurality of patient models with the second directed graph comprises, for each patient model, determining a status of at least one of the plurality of elements of the second directed graph.
22. A system according to clause 20 or clause 21, wherein, for a said patient model, the status of a said element is dependent on availability of data associated with a clinical step which is associated with the said element, in the said patient model.
23. A system according to clause 20 or clause 21, wherein each patient model comprises a plurality of patient entries, each patient entry comprising at least one attribute value, and determining a status of a said element comprises:
   maintaining a first association between at least one of the patient entries of a said patient model and an identifier from a plurality of identifiers;
   maintaining a second association between the said element and the identifier from the plurality of identifiers;
   selecting, based on the first and second association, a said attribute value associated with the said element; and
   determining, based on a comparison of the attribute value associated with the said element to a conditional parameter value associated with the said element, whether the conditional parameter value associated with the said element is satisfied.
24. A system according to any one of clauses 16 to 23, wherein determining which of the directed graphs is a preferred directed graph comprises:
   determining a first measure indicative of an average conformity of the first set of patient models to the first directed graph;
   determining a second measure indicative of an average conformity of the second set of patient models to the second directed graph; and
   performing a comparison of the first plurality of patient outcome measures with the second plurality of patient outcome measures using the first and second measures.
25. A system according to clause 24, wherein the first measure is dependent on an average status for the first set of patient models of the plurality of elements of the first directed graph.
26. A system according to clause 24 or clause 25, wherein the second measure is dependent on an average status for the second set of patient models of the plurality of elements of the second directed graph.
27. A system according to any one of clauses 16 to 26, wherein determining which of the first and second directed graphs is a preferred directed graph comprises:
   transmitting data indicative of a result of the comparison of the first set of patient models with the second set of patient models for receipt by the user device;
   receiving from the user device data indicative of a decision in respect of the result of the comparison; and
   selecting, based on the received data indicative of the decision, one of the first or second directed graphs.
28. system according to clause 18, wherein the at least one memory and computer program code are configured to, with the at least one processor, cause the system to:
   if the first directed graph is the preferred directed graph, transmit data indicative of the first plurality of patient outcome measures for receipt by the user device; or
   if the second directed graph is the preferred directed graph, transmit data indicative of the second plurality of patient outcome measures for receipt by the user device.
29. A computer program comprising a set of instructions, which, when executed by a computerised device, cause the computerised device to perform a method of transmitting healthcare data to a user device, the user device being configured for use in analysing medical information, the method comprising:
   maintaining, in a first database, data representing a first directed graph representing at least part of a medical guideline and a second directed graph representing the at least part of a medical guideline and a modification to the at least part of a medical guideline, each directed graph comprising a respective plurality of elements representing a clinical step;
   maintaining, in a second database, a plurality of patient models each comprising healthcare data associated with a respective patient;
   identifying a first set of the patient models representing patients that have been treated based on the at least part of a medical guideline as represented by the first directed graph and a second set of the patient models representing patients that have been treated based on the at least part of a medical guideline as represented by the second directed graph;
   determining, based on a comparison of the first set of the patient models with the second set of the patient models, which of the first and second directed graphs is a preferred directed graph; and
   responsive to the determination, transmitting data representing the preferred directed graph for receipt by the user device.
30. A method of transmitting healthcare data to a user device, the user device being configured for use in analysing medical information, the method comprising:
   maintaining, in a first database, data representing a first directed graph representing at least part of a medical guideline and a second directed graph representing the at least part of a medical guideline and a modification to the at least part of a medical guideline, each directed graph comprising a respective plurality of elements representing a clinical step;
   maintaining, in a second database, a plurality of patient models each comprising healthcare data associated with a respective patient;
   identifying a first set of the patient models representing patients that have been treated based on the at least part of a medical guideline as represented by the first directed graph and a second set of the patient models representing patients that have been treated based on the at least part of a medical guideline as represented by the second directed graph;
   determining, based on a comparison of the first set of the patient models with the second set of the patient models, which of the first and second directed graphs is a preferred directed graph; and
   responsive to the determination, transmitting data representing the preferred directed graph for receipt by the user device.

## Claims

1. A system (100) operable to transmit healthcare data to a user device (106a-d), the user device (106a-d) being configured for use in analysing medical information, the system (100) comprising at least one processor (102a-n) and at least one memory (104a-n) including computer program code, the at least one memory (104a-n) and computer program code configured to, with the at least one processor (102a-n), cause the system (100) to:
maintain, in a first database (108), data representing a first directed graph (600) representing at least part of a medical guideline, the first directed graph (600) comprising a plurality of elements representing a clinical step;
maintain, in a second database (108), a plurality of patient models each comprising healthcare data associated with a respective patient;
select at least one element from the plurality of elements by processing the plurality of patient models and the data representing the first directed graph (600) to identify at least one element at which treatment of a subset of patients has deviated from the at least part of a medical guideline;
identify, based on a combination of the at least one selected element and the plurality of patient models, a first patient cohort whose treatment has deviated from the at least part of a medical guideline at the at least one selected element and a second patient cohort whose treatment has conformed to the at least part of a medical guideline at the at least one selected element;
process the plurality of patient models representing the first and second patient cohorts to identify at least one patient cohort characteristic distinguishing the first patient cohort from the second patient cohort;
generate a second directed graph (610, 620) dependent at least on the at least one identified patient cohort characteristic; and
transmit data representing the second directed graph (610, 620) for receipt by the user device (106a-d).

2. A system (100) according to claim 1, wherein selecting the at least one element comprises processing the plurality of patient models and the data representing the first directed graph (600) to identify at least one element at which the subset of patients whose treatment has deviated from the at least part of the medical guideline exceeds a predetermined proportion of the patients associated with the plurality of patient models.

3. A system (100) according to claim 1 or claim 2, wherein the at least one patient cohort characteristic comprises at least one of:
an age;
a height;
a weight;
a sex;
a body mass index;
a genetic mutation;
an associated medical practitioner; and
a location.

4. A system (100) according to any preceding claim, wherein the memory (104a-n) and computer program code are configured to, with the at least one processor (102a-n), cause the system (100) to override the selection of the at least one element based on a user input.

5. A system (100) according to any preceding claim, wherein the at least one selected element is associated with at least one conditional parameter value, the patient models each comprise a plurality of patient attribute values corresponding to respective clinical steps, and the first and second patient cohorts are identified based on a comparison of the at least one conditional parameter value with respective patient attribute values from the plurality of patient models.

6. A system (100) according to any of claims 1 to 4, wherein the first and second patient cohorts are identified based on an availability of healthcare data in the respective patient models corresponding to the at least one selected element.

7. A system (100) according to any preceding claim, wherein processing the plurality of patient models representing the first and second patient cohorts to determine at least one patient cohort characteristic distinguishing the first patient cohort from the second patient cohort comprises processing the plurality of patient models using at least one of:
principal component analysis;
random forest regression; and
regularized regression.

8. A system (100) according to any preceding claim, wherein the second directed graph (610, 620) comprises an indication that the clinical step represented by the at least one selected element is not recommended for patients associated with the at least one identified patient cohort characteristic.

9. A system (100) according to claim 8, wherein the second directed graph (620) comprises:
a plurality of nodes (606a-e);
a set of directed edges; and
at least one further node (606f) connected to the selected element,
wherein the at least one further node (606f) comprises the indication that a clinical step represented by the selected element is not recommended for patients associated with the at least one identified patient cohort characteristic.

10. A system (100) according to claim 9, wherein the second directed graph (620) comprises at least one further directed edge connected at a first end to the further node (606f), the further directed edge being indicative of a deviation from the at least part of a medical guideline for the first patient cohort.

11. A system (100) according to any preceding claim, wherein the at least one memory (104a-n) and computer program code are configured to, with the at least one processor (102an), cause the system (100) to:
based on a user input indicative of a decision in respect of patients associated with the at least one identified patient cohort characteristic, modify the second directed graph (610, 620) .

12. A system (100) according to claim 9 or claim 10, wherein the at least one memory (104a-n) and computer program code are configured to, with the at least one processor (102an), cause the system (100) to:
maintain, in a third database (108), a further patient model comprising healthcare data associated with a patient, the patient being associated with the at least one identified patient cohort characteristic;
determine, based on a combination of the second directed graph (620) and the further patient model, a status of the at least one further node (606f) and/or a status of a directed edge 607h connected thereto; and
dependent on the status of the at least one further node (606f) and/or the status of the directed edge 607h connected thereto, transmit data indicative of the status of the at least one further node (606f) and/or the status of the directed edge 607h connected thereto for receipt by the user device (106a-d), the data indicative of the status of the at least one further node (606f) and/or directed edge 607h connected thereto being for use in determining whether a clinical step represented by the selected node (606c) is not recommended for the patient.

13. A system (100) according to claim 12, wherein the at least one memory (104a-n) and computer program code are configured to, with the at least one processor (102a-n), cause the system (100) to:
dependent on the status of the further node (606f) and/or the status of the directed edge connected thereto, transmit data indicative of a request for input from a user of the user device (106a-d) for receipt by the user device (106a-d);
receive, from the user device (106a-d), data indicative of further clinical steps to be represented by a further plurality of nodes; and
modify the second directed graph (610, 620) based on the received data indicative of the further clinical steps.

14. A computer program comprising a set of instructions, which, when executed by a computerised device, cause the computerised device to perform a method of transmitting healthcare data to a user device (106a-d), the user device (106a-d) being configured for use in analysing medical information, the method comprising:
maintaining, in a first database (108), data representing a first directed graph (600) representing at least part of a medical guideline, the first directed graph (600) comprising a plurality of elements representing a clinical step;
maintaining, in a second database (108), a plurality of patient models each comprising healthcare data associated with a respective patient;
selecting at least one element from the plurality of elements by processing the plurality of patient models and the data representing the first directed graph (600) to identify at least one element at which treatment of a subset of patients has deviated from the at least part of a medical guideline;
identifying, based on a combination of the at least one selected and the plurality of patient models, a first patient cohort whose treatment has deviated from the at least part of a medical guideline at the at least one selected element and a second patient cohort whose treatment has conformed to the at least part of a medical guideline at the at least one selected element;
processing the plurality of patient models representing the first and second patient cohorts to determine at least one patient cohort characteristic distinguishing the first patient cohort from the second patient cohort;
generating a second directed graph (610, 620) dependent at least on the at least one identified patient cohort characteristic; and
transmitting data representing the second directed graph (610, 620) for receipt by the user device (106a-d).

15. A method of transmitting healthcare data to a user device (106a-d), the user device (106a-d) being configured for use in analysing medical information, the method comprising:
maintain, in a first database (108), data representing a first directed graph (600) representing at least part of a medical guideline, the first directed graph (600) comprising a plurality of elements representing a clinical step;
maintaining, in a second database (108), a plurality of patient models each comprising healthcare data associated with a respective patient;
selecting at least one element from the plurality of elements by processing the plurality of patient models and the data representing the first directed graph (600) to identify at least one element at which treatment of a subset of patients has deviated from the at least part of a medical guideline;
identifying, based on a combination of the at least one selected element and the plurality of patient models, a first patient cohort whose treatment has deviated from the at least part of a medical guideline at the at least one selected element and a second patient cohort whose treatment has conformed to the at least part of a medical guideline at the at least one selected element;
processing the plurality of patient models representing the first and second patient cohorts to determine at least one patient cohort characteristic distinguishing the first patient cohort from the second patient cohort;
generating a second directed graph (610, 620) dependent at least on the at least one identified patient cohort characteristic; and
transmitting data representing the second directed graph (610, 620) for receipt by the user device (106a-d).
